# EUROPEAN PATENT APPLICATION

(11) **EP 2 319 414 A1**
(43) Date of publication of application: **11.05.2011**
(21) Application number: 10188842.8
(22) Date of filing: 26.10.2010
(51) Int. Cl.: A61B 6/00, G01N 21/64

(54) **Wound Goggles**

(30) Priority: 10.11.2009 US 615950
(71) Applicant: THE OHIO STATE UNIVERSITY RESEARCH FOUNDATION, Columbus OH 43212-1154 (US)
(72) Inventor: Sen, Chandan, K., Ohio 43220 (US); Xu, Ronald X., Ohio 43228 (US)
(74) Representative: Boyce, Conor

(57) **Abstract**

Appropriate assessment of wound oxygenation is critical to establish a diagnosis, to monitor the effect of the treatment, to guide the therapeutic process, to identify the presence of infection, and to predict the treatment outcome. Embodied systems and methods represent an enabling technology for noninvasive and objective assessment of wound tissue oxygenation. In addition to wound healing, disclosed embodiments offer low cost and portable avenues for noninvasive assessment of multiple clinical parameters for the detection and intervention of various malignancies in multiple soft tissue systems.

## Description

### TECHNICAL FIELD

The disclosed embodiments of the present invention are in the field of non-invasive systems for multi-spectral medical imaging, particularly a wearable goggle system for quantitative wound imaging.

### BACKGROUND OF THE ART

Existing methods for tissue oxygenation measurement are not appropriate for wound tissue imaging because of several technical limitations. First of all, many oximetry devices measure tissue oxygen tension (i.e., PO2, the partial pressure of the dissolved oxygen molecules) instead of oxygen saturation (i.e., StO2, the amount of oxygen bound to hemoglobin as a percentage of the maximal binding capability). These devices include polarographic O2 microelectrode, luminescence-based optical sensors, Electron Paramagnetic Resonance spectroscopy (EPRs), transcutaneous oxygen pressure meter (TcPO2), and ¹⁹F NMR spectroscopy. Although tissue PO2 and StO2 are correlated by the well established dissociation curve, the correlation is effected by multiple physiologic parameters. Second, blood oxygen level-dependent (BOLD) MR imaging measures tissue oxygenation, but is only sensitive to deoxygenated hemoglobin. Besides, it represents the significantly added cost and complexity. Third, the emerging technique of near infrared (NIR) diffuse optical imaging and spectroscopy (DOIS) detects multiple tissue parameters such as StO2, total hemoglobin concentration (Hbt), water, and lipid. However, a typical NIR DOIS device requires the surface contact between the sensor head and the biologic tissue. The measurement has low spatial resolution and is heavily affected by background scattering of deep tissue.

In recent years, hyperspectral imaging has been used for wound oxygen assessment. Hyperspectral imaging capture the reflectance images of the wound at multiple wavelengths, and reconstruct tissue oxygen saturation based on the characteristics reflectance spectra of oxy and deoxyhemoglobin. Clinical studies have demonstrated the value of hyperspectral imaging in the identification of microvascular abnormalies and tissue oxygenation associated with diabetic foot and the capability to predict the ulcer healing. However, the performance of current hyperspectral imaging systems is not satisfactory because of the measurement variations due to skin pigmentation, tissue density, lipid content, and blood volume changes. Furthermore, current systems are not portable, and therefore, are illsuited for intra-operative and/or military environments.

### SUMMARY

This and other unmet needs of the prior art are met by the system and method as described in more detail below. Embodied systems represent an enabling technology for noninvasive and objective assessment of wound tissue oxygenation. Appropriate assessment of wound oxygenation is critical to establish a diagnosis, to monitor the effect of the treatment, to guide the therapeutic process, to identify the presence of infection, and to predict the treatment outcome. In addition to wound healing, disclosed embodiments offer low cost and portable avenues for noninvasive assessment of multiple clinical parameters for the detection and intervention of various malignancies in multiple soft tissue systems.

An exemplary embodiment includes a system for visual wound assessment on a subject tissue, comprising:
an illumination unit adapted to emit an excitation light on the subject tissue;
an eyewear unit comprising:
   an imaging unit to collect light from the subject tissue; and
   an image display unit for displaying a hyperspectral image; and a control unit operably connected to the eyewear unit and the illumination unit, the control unit comprising:
      a light processing module;
      an imaging fiber guide to transport emitted light received by the imaging unit to the light processing module; and
      a computer programmed to:
         synchronize activities of the illumination unit, the imaging unit, the light processing module, and the image display unit; and
         generate the hyperspectral image in about real time.

In at least one exemplary embodiment, at least a portion of the control unit is contained in a portable backpack.

The imaging unit of an exemplary embodiment comprises an automated zoom lens. The light processing module of an exemplary embodiment comprises a CCD camera equipped with a liquid crystal tunable filter and an optical adaptor. The light processing module may further comprises a polarizer.

The illumination unit of an exemplary embodiment comprises, in alignment a spectrally tunable light source and a programmable mirror galvanometer adapted to receive and direct excitation light in a point-to-point scanning pattern. The illumination unit of various exemplary embodiments may comprise, in alignment, a spectrally tunable light source, a polarizer, an optical chopper; and a shutter. In alternative full-field embodiments, the illumination unit may comprise, in alignment, a tunable light source; and a lens assembly positioned to expand the light for full-field illumination of the subject tissue. In various embodiments, the illumination unit may be positioned to provide the excitation light at an oblique angle relative to the subject tissue.

In various embodiments the system may be adapted to include 3D multi-view, multi-spectral imaging capability by positioning a cylindrical mirror between the imaging unit and the *in vivo* tissue of interest.

Embodiments include an arrangement for visual wound assessment on a subject tissue, comprising:
an illumination unit adapted to emit an excitation light on the subject tissue, the illumination unit comprises in alignment:
   a tunable light source; and
   a programmable mirror galvanometer adapted to receive and
   direct excitation light in a point-to-point scanning pattern.
an imaging unit comprising an automated zoom lens to collect light from the subject tissue;
an image display unit for displaying a hyperspectral image; and
a control unit operably connected to the illumination unit, the imaging unit, and the image display unit, the control unit comprising:
   a light processing module comprising a CCD camera equipped with a liquid crystal tunable filter and an optical adaptor;
   an imaging fiber guide to transport emitted light received by the imaging unit to the light processing module; and
   a computer programmed to:
      synchronize activities of the illumination unit, the imaging unit, the light processing module, and the image display unit; and
      generate the hyperspectral image in about real time.

Embodied arrangements may include an eyewear unit incorporating the illumination unit, the imaging unit, and the image display unit. The embodiments may also include a cylindrical mirror positioned between the eyewear unit and the subject tissue. In various embodiments, the illumination unit is positioned to provide the excitation light at an oblique angle relative to the subject tissue. The illumination step may occur via a point-to-point scanning pattern in preferred arrangements.

An exemplary embodiment comprises a wearable goggle system for quantitative assessment and/or visualization of wound tissue oxygenation on a subject tissue. The system comprises of an eyewear unit, a spectrally tunable illumination unit, and a control unit. The eyewear unit comprises an imaging unit. In a preferred embodiment, the imaging unit comprises automated zoom lenses for receiving emitted and/or reflected light from at least a portion of a subject tissue. Exemplary embodiments comprise an imaging fiber guide to transport emitted light received by the zoom lenses to the light processing module. The eyewear unit further comprises a head mounted image display unit for displaying a generated image. The spectrally tunable excitation illumination unit is used to illuminate at least a portion of the subject tissue. In a preferred embodiment, a mirror galvanometer may be included for point-to-point scanning illumination instead of, or in addition to, full-field illumination of the subject tissue. A control unit is operably connected to the eyewear unit and the illumination unit. The control unit comprises a light processing module comprising a CCD camera equipped with a tunable filter and an optical adaptor. Additionally, the control unit of an exemplary embodiment comprises a computer programmed to synchronize tissue illumination, filter configuration, lens control, data acquisition, and image display.

In operation, an exemplary embodiment emits excitation light from a spectrally tunable illumination unit. The light is modulated and collimated for pulsed illumination on the surgical site. Diffuse reflectance is collected by automated zoom lenses and delivered through high resolution imaging fiber guides to the control unit. The control unit may house the image acquisition and processing module components of the system.

In at least one embodiment, the control unit may be specifically designed as a portable backpack unit. Such a backpack unit would be a compact image acquisition and processing module that would allow unlimited and unrestricted mobility to the clinician and/or to a medic in a militarized zone. To minimize the weight of the backpack unit, a single high resolution, high sensitivity CCD camera with custom designed view separation optics similar to that in color photography may be used for simultaneous acquisition of background and fluorescence images from two or more fiber bundles. The above components (i.e., light source, motorized zoom lenses, HMD, and CCD camera) may be connected to a computer with embedded programs for real time control and synchronization of illumination, automatic focus, image acquisition, processing, and HMD display.

Exemplary embodiments also include methods for visual wound assessment. The exemplary methods include the steps of, providing an eyewear unit, an illumination unit, and a control unit; illuminating a subject tissue with excitation light at multiple wavelengths; receiving light emitted from the subject tissue; processing the emitted light to generate a processed hyperspectral image comprising reflectance spectra data for oxy and deoxy hemoglobin; and displaying the processed hyperspectral image comprising reflectance spectra data for oxy and deoxy hemoglobin in about real time onto a head mounted image display unit positioned on the eyewear unit.

### BRIEF DESCRIPTION OF THE DRAWINGS

A better understanding of the exemplary embodiments of the invention will be had when reference is made to the accompanying drawings, wherein:

**FIGURE 1** is a schematic demonstrating an exemplary embodiment of the wearable goggle system.

**FIGURE 2** is a schematic illustrating the eyewear unit of an exemplary embodiment.

**FIGURE 3** is a schematic showing the control unit of an exemplary embodiment.

**FIGURE 4** is a schematic illustrating the illumination unit of an exemplary embodiment.

**FIGURE 5A** is a schematic showing an embodiment with full field illumination. **FIGURE 5B** is a schematic showing an embodiment with point to point scanning illumination.

**FIGURE 6A** is a depiction of an alternative embodiment for 3D wound margin assessment using a cylindrical mirror. **FIGURE 6B** is a schematic demonstrating 3D multi-view, multi-spectral imaging using a cylindrical mirror. clinical application of an exemplary embodiment.

**FIGURE 7** is a schematic demonstrating clinical application of an exemplary embodiment.

### DETAILED DESCRIPTION

As used herein, the phrase "operably connected" is intended to mean coupled or connected, either directly or indirectly, such that the connected structures are operable to perform a desired function.

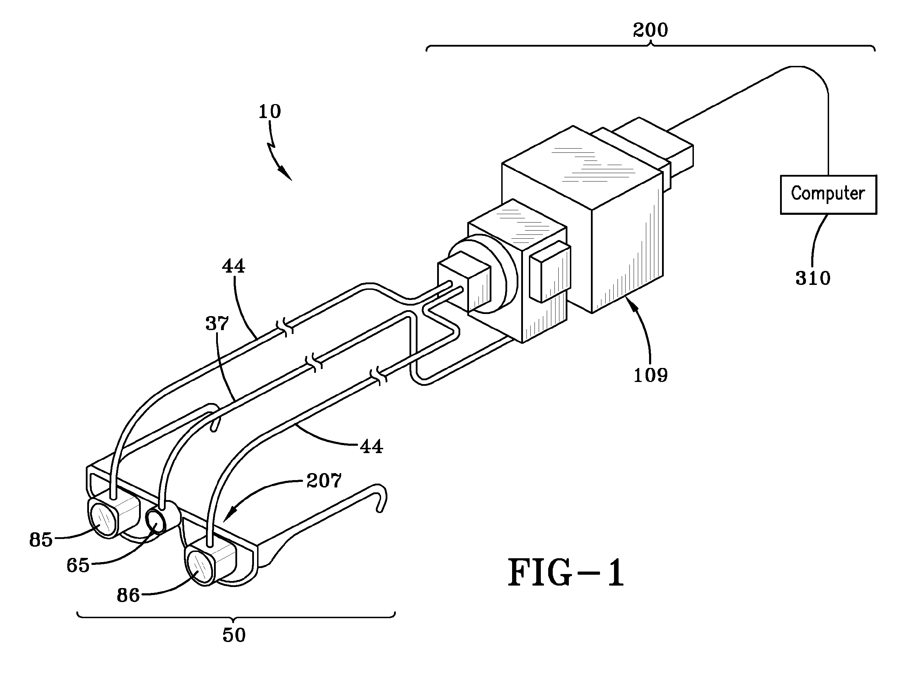

Fig. 1 shows an exemplary embodiment of the wound goggle system **10** for quantitative assessment and/or visualization of wound tissue oxygenation of a subject tissue. The wearable goggle system **10** comprises an eyewear unit **50,** an illumination unit **65,** and a control unit **200.** The eyewear unit **50** of an exemplary embodiment comprises one or more imaging units **85, 86.** Preferably, imaging units **85, 86** comprises automated zoom lenses for receiving emitted and/or reflected light from at least a portion of a subject tissue. To display the generated image, the eyewear unit **50** further comprises a head mounted image display (HMD) unit **207,** for example, a Personal Cinema System by Head Play Inc. (Los Angeles, CA).

The control unit **200** may comprise a light processing module **109** and a computer **310.** The control unit **200** may be operably connected to the eyewear unit **50** and the illumination unit **65** via a cable **37.** Alternatively, the control unit **200** may be wirelessly connected to the eyewear unit **50** and/or the illumination unit **65.** In various exemplary embodiments, the illumination unit **65,** imaging units **85,86,** HMD **207,** and the light processing module **109** are connected to the computer **310.** The computer **310** may comprise a commercial vision system, for example, a compact vision system (CVS) from National Instrument (Austin, Texas), with embedded programs for real time control and synchronization of illumination, automatic focus, image acquisition, processing, and image display. In at least one embodiment, at least a portion of the control unit **200** may be specifically designed as a portable backpack unit **110.** Such a backpack unit **110** would comprise a compact image acquisition and processing module that would allow unlimited and unrestricted mobility to the clinician.

In operation, excitation light from a spectrally tunable illumination unit **65** may be modulated and collimated for pulsed illumination on the surgical site. Diffuse reflectance is collected by automated zoom lenses within the imaging unit **85, 86** and delivered through high resolution imaging fiber guides **44** to the control unit **200.** The control unit **200** may house the image acquisition and processing module components of the system.

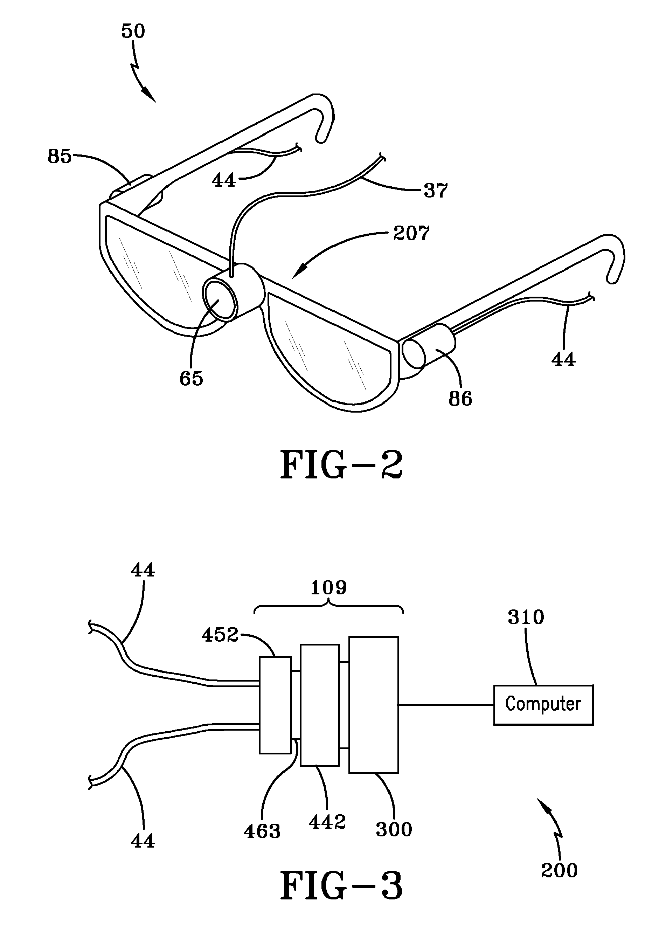

Fig. 2 shows an exemplary embodiment of the eyewear unit **50.** In this embodiment, two imaging units **85,86** may be positioned parallel to the axis of the image display **207** of eyewear unit **50.** Imaging units **85,86** include motorized zoom lenses. High resolution imaging fiber guides **44** connect the imaging units **85,86** with the control unit (not shown in Fig. 2). Collected images are transferred to the control unit **200** for multi-spectral processing. Eyewear unit **50** includes a head mounted image display unit **207** adapted to display a generated image. Eyewear unit **50** also includes a spectrally tunable excitation illumination unit **65.** The illumination unit **65** is adapted to illuminate at least a portion of the subject tissue. The illumination unit 65 is connected to the control unit (not shown) via cable **37.**

Referring to Fig. 3, the control unit **200** includes a light processing module **109.** Direct incident light or light delivered from imaging fibers **44** is received into the light processing module **109.** Preferably, the light processing module **109** comprises a CCD camera **300** equipped with a liquid crystal tunable filter **442** and an optical adaptor **452.** The CCD camera **300** may comprise view separation optics similar to that used with color photography that allow simultaneous acquisition of background and fluorescence images from if two or more fiber bundles **44 are** used as shown. The light processing module **109** may further comprise a polarizer/analyzer **463,** which may be used to reduce specular reflection and to study tissue polarization characteristics. The control unit **200** further comprises a computer **310** programmed with an imaging acquisition module. In an exemplary embodiment, the computer **310** is programmed to synchronize tissue illumination, filter configuration, lens control, data acquisition, and image display. The control unit **200** also synchronizes the controls and synchronizes the light processing module **109.** High resolution imaging fiber guides **44** transport emitted light received by the zoom lenses (not shown) to a light processing module **109.** In an alternative embodiment, a miniature monochrome CCD camera may be positioned on the eyewear unit, eliminating the need for fiber guides **44.**

An exemplary embodiment may comprise an image processing toolkit for near real-time co-registration, segmentation and visualization of the oxygen map and hypoxic margins. In at least one embodiment, image analysis may be performed using Insight Segmentation and Registration Toolkit (ITK) (National Library of Medicine's Office of High Performance Computing and Communication (HPCC)). ITK is a C++ library and has a wide range of functionalities for registration and segmentation and provides viable interfaces for other tasks such as reconstruction and visualization. To provide an immersive visual environment with minimal interference to the surgical procedure, an exemplary embodiment may integrate basic functions of ITK into the LabVIEW Real-Time environment (National Instrument) through DLLs.

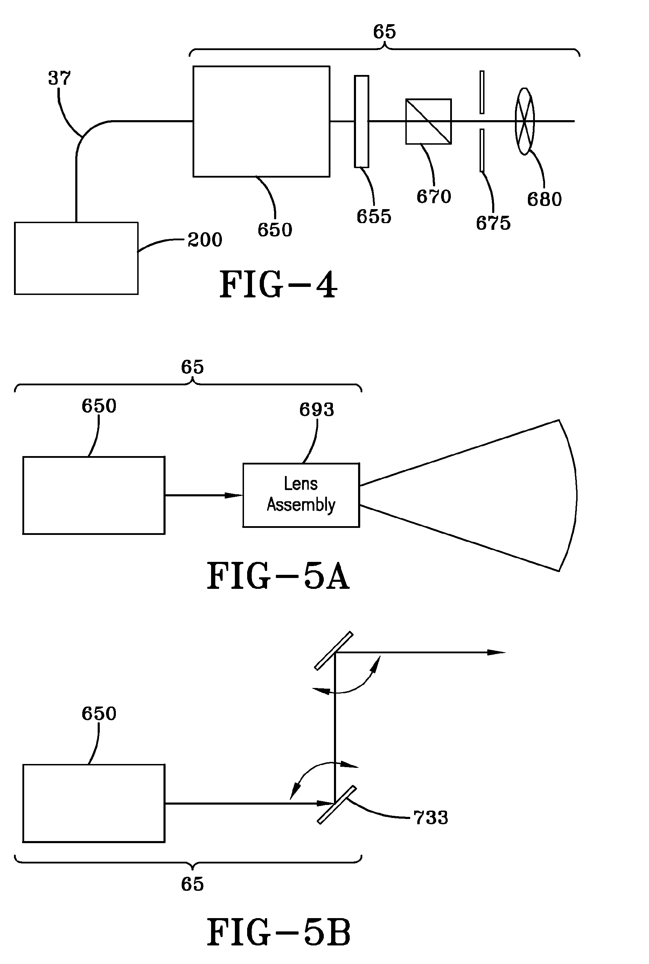

Referring to Fig. 4, the illumination unit **65** may be connected to control unit **200** via cable **37.** Alternatively, illumination unit **65** may be connected to control unit **200** via a wireless connection (not shown). The illumination unit **65** may comprise a tunable light source **650,** a polarizer **655,** a beam splitter **670,** an optical chopper **675,** and a shutter **680.** In an exemplary embodiment, visible light from a digital light processing (DLP) based tunable light source **650** and will be polarized. The exiting light may be split if a beam splitter **670** is included. Before exiting through iris shutter **680,** the light may be modulated with an optical chopper **675.**

Embodiments may operate in the various illumination modes: (1) hyperspectral or multispectral imaging mode with full-field broadband illumination, (2) point-to-point scanning mode without full-field background illumination, (3) combination of full-field illumination and point-to-point scanning within a specific region of interest. In various embodiments, the illumination unit **65** may generate light at multiple wavelengths in sequence. The tunable light source **650** may be positioned on the eyewear, or alternatively, may be located on the control unit. If the tunable light source **650** is located on the control unit it may be directed to by fiber optic cables to illuminate the desired area of interest.

Referring to Fig. 5A, in embodiments where illumination unit **65** provides full field illumination, multi-wavelength light is emitted from a tunable light source **650** (e.g., light emitting diodes (LEDs), Laser Diodes (LDs), optical fibers, etc.). The emitted light may be directed through a lens assembly **693** before illuminating the desired field. In this way, the entire wound or a portion thereof may be evenly illuminated if desired, such that the diffuse reflectance image may be captured at the same time.

However, the illumination mode depicted in Fig. 5A may not be appropriate for particular wound imaging applications because of the following reasons. First, since light is multi-scattered in biologic tissue, simultaneous illumination of multiple heterogeneous regions will introduce crosstalk and eventually degrade the accuracy and the sensitivity of regional tissue oxygen measurements. Second, simultaneous illumination of multiple regions will significantly increase the background signal intensity and put heavy load to the camera system. In the case of imaging the wound tissue with large absorption contrast (for example, necrosis surrounded by normal tissue), an appropriate illumination condition may not be achievable because of the difficult to balance between the over-exposure of the surrounding tissue and the under-exposure of the necrosis. Over-exposure will saturate the camera while under-exposure will force the camera to work at the low end of dynamic range.

Referring to Fig. 5B, various embodiments overcome these limitations using a point-to-point scanning light source. In embodiments where illumination unit **65** provides point-to-point scanning, multi-wavelength light from a tunable light source **650** (e.g., light emitting diodes (LEDs), Laser Diodes (LDs), optical fibers, etc.) may be modulated using an optical chopper (not shown) and steered using a mirror galvanometer **733** to rapidly scan the region of interest, preferably following a programmed pattern. In these embodiments, the illumination intensity and the camera exposure can be optimized at the area of interest. A polarizer/analyzer positioned on the light processing unit **65** may reduce specular reflection and to study tissue polarization characteristics. Reflected light from the superficial tissue layers will pass a liquid crystal tunable filter (LCTF) and collected by a CCD camera.

Depending on the specific clinical application, these operational modes may be used interchangeably. For example, to image the dark necrotic region surrounded by tissue of less absorption, the combinational imaging mode may be used. In this example, the necrotic region will be scanned point-by-point to increase the measurement sensitivity while the full-field illumination may provide the oxygen map of the surrounding tissue. In an exemplary embodiment, a programmable galvanometer is used to facilitate custom design of the scanning pattern for maximal imaging quality within the region. The programmable galvanometer permits custom design of the scanning pattern for maximal imaging quality within the region of interest. In addition, the system can also be used for other applications such as fluorescence imaging. Since the scanning speed of the galvanometer is greater than 500µs/line and the diffuse reflectance patterns at multiple scanning points of light can be captured by a single snap shot of the CCD camera with the designated exposure time, the expected sampling rate for this proposed imaging system will be comparable with that of existing wound imaging systems.

Exemplary embodiments may utilize optical scanning mechanisms similar to those which have been successfully implemented in many commercial products such as bar code readers. Current technical advances in optical scanning and camera systems allow for near real-time image acquisition rate in these point-to-point systems.

Exemplary embodiments may employ quantitative methods for diffuse optical measurements of tissue oxygenation (StO2) and hemoglobin concentration (Hbt) which have been previously described (see e.g., Refs *20-23*). Additionally, tissue oximeter prototypes have been previously validated through benchtop tests, animal studies, and clinical trials (Ref: *24-27*). The quantitative methods for tissue oxygen measurement have been tested in multiple clinical applications, including breast cancer detection and peripheral vascular disease (PVD) diagnosis (Refs*: 15, 25, 28*). Furthermore, tissue StO2 measurements were integrated with oxygen tension (PO2) and blood flow measurements for quantitative evaluation of tumor tissue oxygen metabolism.

An exemplary embodiment may utilize an integrated system consisting of a near infrared tissue oximeter for StO2 and Hbt measurements, a laser Doppler for blood flow (BF) measurement, and an electron paramagnetic resonance spectroscope (EPRS) for oxygen tension (PO2) measurement. The strategy of simultaneous quantitative measurement of multiple tissue parameters permits detection and analysis of oxygen transport deficiencies in wound tissue which will facilitate accurate diagnosis and efficient treatment.

To determine a resection boundary, an exemplary embodiment may utilize various image segmentation techniques including both color and texture segmentation to classify the pixels into different structures. The initial boundary of the region of interests or structures may be obtained from a color segmentation algorithm followed by morphological operations. The boundaries will be further refined and smoothed using elastic deformable techniques (i.e., active contour, available in ITK). A closed elastic curve is then fitted and grown by suitable deformations so that the curve encloses homogenous regions.

Given the dynamic nature of the acquisition, the fitted region is obtained for one of the first acquired images. For consecutively acquired images, the resection boundaries may be determined based on the active contour model with the input of previously fitted region to minimize the computational cost (See Refs: 30-32). In addition, in order to avoid effects of changes in illumination and view angles, temporal filtering among multiple consecutive frames and periodic reinitialization may be carried out.

Various image analysis systems and algorithms previously developed for processing large image sets with terabytes of data have been previously described. Specific embodiments leverage and extend the systems above to achieve near real-time image analysis and display. Specifically, fast color segmentation algorithms may be used to initialize the boundary detection. Subsequently, the C++ implementations of the active contour algorithm in ITK may be used to achieve efficient boundary tracking. For image co-registration, two approaches may be used. First, extrinsic calibration may be carried out for the cameras for different modalities. This will provide an initial estimate of the transformation between the images. Next, color-based fast segmentation of landmarks may be used on the tissue to enable quick feature matching for fine tuning of the affine transformation between different images.

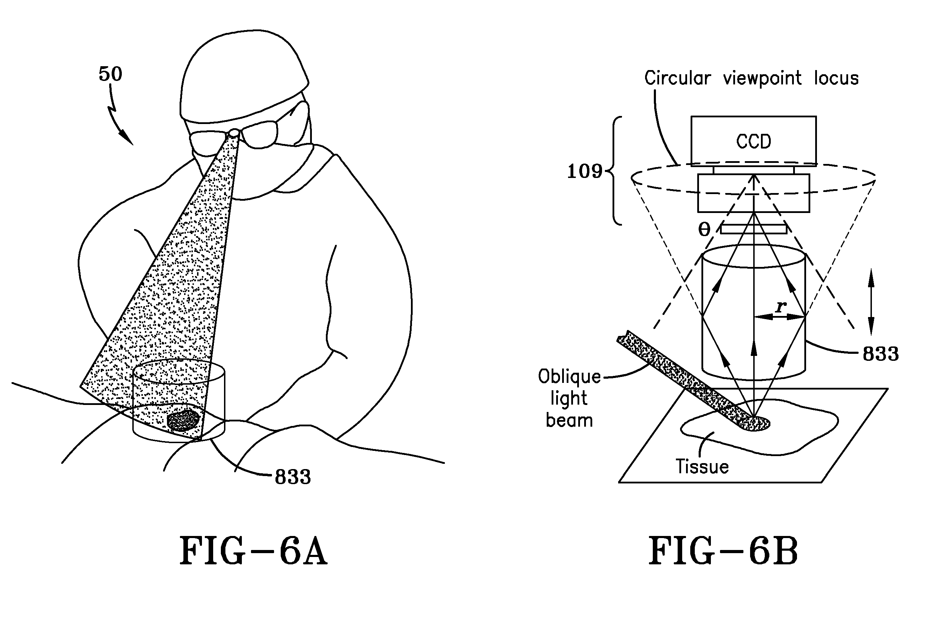

Referring to the exemplary embodiment shown in Fig. 6, the goggle system may be adapted to include 3D multi-view, multi-spectral imaging capability by positioning a cylindrical mirror **833** between the proposed multi-spectral imaging system and the *in vivo* tissue of interest, as shown in Fig 6A.

Referring to Fig. 6B, a typical snapshot of the CCD camera includes a top view image of the tissue at the center, surrounded by image segments from multiple view points. As represented in Fig 6B, the multiple view points are due to the reflection of light by the inner surface of the cylindrical mirror. The mapping between the sample space and the cylindrical mirror space will be calibrated in advance. The field of view is defined as Φ = tan⁻¹[2cosθ sin² θ /(sinθ + 2sinθ cos² θ )] , where θ is the half field of view. The tangential resolution is defined as the ratio between a circle of pixels of radius ρᵢ on the image plane and a circle of radius ρ*ₛ* on the scene plane: ρ*ᵢ* / ρ*ₛ* = ρ*ᵢ* sinθ /(2 sinθ - wρ*ᵢ* sinθ ), where r is the radius of the cylindrical mirror and *w* is the depth of the scene. The above radical imaging system can be used to estimate the diffusion and reflection parameters of an analytic bidirectional reflectance distribution function (BRDF) model.

The 3D multi-view, multi-spectral radical imaging system of an exemplary embodiment facilitates several techniques for quantitative wound margin assessment: (1) 3D images acquired at multiple wavelengths to reconstruct and document wound oxygen distribution in 3D; (2) by moving the cylindrical mirror in the axial direction, it is possible to obtain a large number of multi-view images for improved spatial (especially depth) resolution of the reconstructed wound images; (3) when the sample is illuminated by an obliquely incident light beam, the multi-view, multi-spectral radical imaging system can capture the diffuse reflectance profiles from all the view angles, allowing for accurate reconstruction of regional tissue absorption and scattering properties. Continuous scanning of the light beam at different wavelengths will generate a 3D map of tissue optical properties and oxygenation distribution.

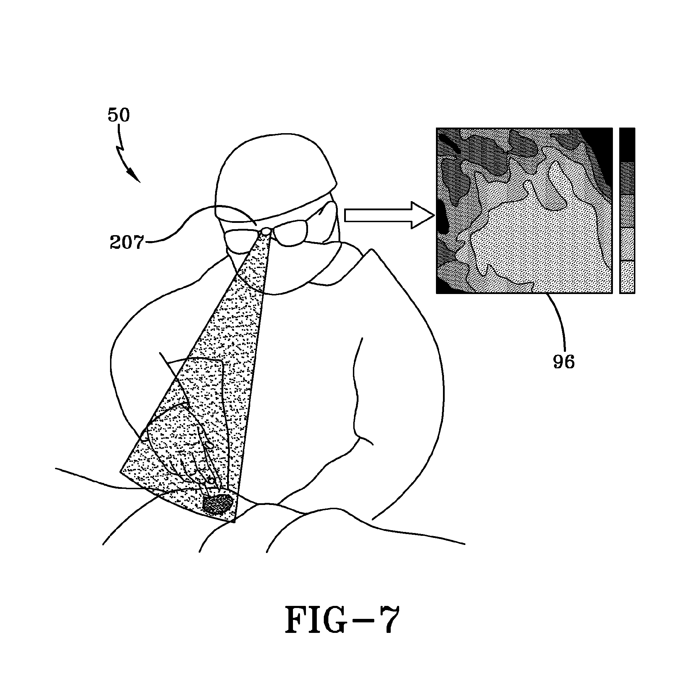

Referring to Fig. 7, exemplary embodiments include methods for intra-operative visual wound assessment. An exemplary method includes the steps of: providing a wearable goggle system comprising an eyewear unit, a spectrally tunable light source, and a control unit; illuminating a subject tissue with excitation light of multiple wavelengths; receiving diffuse reflected light emitted from the subject tissue; processing the emitted light to generate a processed image **96** comprising reflectance spectra data for oxy and deoxy hemoglobin; and displaying the processed image **96** in near real time onto a head mounted image display **207** in the eyewear unit **50.**

### PUBLICATIONS

The following references may provide exemplary procedural and other details supplementary to those set forth herein:
1. A. J. Singer, R. A. Clark, N Engl J Med 341, 738 (Sep 2, 1999).
2. H. Brem et al., Mol Med 13, 30 (Jan-Feb, 2007).
3. C. K. Sen, Wound Repair Regen 17, 1 (Jan-Feb, 2009).
*4.* US Surgery 6 (2008).
5. G. M. Gordillo et al., Methods Enzymol 381, 575 (2004).
6. F. T. Padberg, T. L. Back, P. N. Thompson, R. W. Hobson, 2nd, J Surg Res 60, 365 (Feb 1, 1996).
7. B. Gallez, C. Baudelet, B. F. Jordan, NMR Biomed 17, 240 (Aug, 2004).
8. R. P. Mason et al., Adv Exp Med Biol 530, 19 (2003).
9. F. Gottrup, R. Firmin, N. Chang, W. H. Goodson, 3rd, T. K. Hunt, Am J Surg 146, 399 (Sep, 1983).
10.A. Scheffler, H. Rieger, Vasa 21, 111 (1992).
11. M. C. Krishna, S. Subramanian, P. Kuppusamy, J. B. Mitchell, Semin Radiat Oncol 11, 58 (Jan, 2001).
12. R. X. Xu, S. P. Povoski, Expert Rev Med Devices 4, 83 (Jan, 2007).
13. L. Khaodhiar et al., Diabetes Care 30, 903 (Apr, 2007).
14.J. Xu, R. Xu, K. Huang, S. Gnyawali, C. K. Sen, Annual conference of Biomedical Engineering Society (2008).
15. X. Cheng et al., Clin Hemorheol Microcirc 31, 11 (2004).
16. S. L. Jacques, J. C. Ramella-Roman, K. Lee, J Biomed Opt 7, 329 (Jul, 2002).
17. N. Ghosh, S. K. Majumder, H. S. Patel, P. K. Gupta, Opt Lett 30, 162 (Jan 15, 2005).
18. L.-H. Wang, S. L. Jacques, L. Zheng, Computer Methods and programs in Biomedicine 47, 131 (1995).
19. R. Xu, A. Rana, Proc. SPIE 6086, 353 (2006).
20.X. Cheng, X. Xu, S. Zhou, L. Wang. (Photonify Technologies, Inc., USA, 2003) pp. 6.
21.X. Cheng *et al.* (Photonify Technologies, Inc., International, 2002).
22. R. X. Xu, B. Qiang, J. J. Mao, S. P. Povoski, Appl Opt 46, 7442 (Oct 20, 2007).
23. X. Cheng et al., Proc. SPIE 4244, 468 (2001).
24. X. Xu et al., Proc. SPIE 4955, 369 (2003).
25. R. X. Xu, D. C. Young, J. J. Mao, S. P. Povoski, Breast Cancer Res 9, R88 (2007).
26. X. Cheng, X. Xu, Proc. SPIE 4955, 397 (2003).
27. B. Wang, S. Povoski, X. Cao, D. Sun, R. Xu, Applied Optics 47, 3053 (June 1 st, 2008).
28. R. X. Xu, J. Ewing, H. El-Dahdah, B. Wang, S. P. Povoski, Technol Cancer Res Treat 7, 471 (Dec. 2008).
29. B. Qiang et al., International Conference on Infrared and Millimeter Waves 31, 248 (2006).
30. L. lbanze, W. Schroeder, The ITK Software Guide 2.4 (Kitware, Inc., 2005), pp.
31. I. Bankman, Handbook of Medical Imaging: Processing and Analysis (Academic Press, 2000), pp.
32. R. F. Chang et al., Ultrasound Med Biol 29, 1571 (Nov, 2003).
33. F. Janoos et al., Med Image Anal 13, 167 (Feb, 2009).
34. K. Mosaliganti et al., IEEE Trans Vis Comput Graph 14, 863 (Jul-Aug, 2008).
35. K. Mosaliganti et al., Med Image Anal 13, 156 (Feb, 2009).
36. K. Mosaliganti et al., IEEE Trans Med Imaging 26, 1283 (Sep, 2007).
37. K. Mosaliganti et al., J Biomed Inform 41, 863 (Dec, 2008).
38.A. Ruiz, M. Ujaldon, L. Cooper, K. Huang, Journal of Signal Processing Systems 55, 229 (2009).
39. R. Xu, B. Qiang, C. Roberts, Proc. SPIE 6138, 363 (2006).
40. D. J. Cuccia, F. Bevilacqua, A. J. Durkin, B. J. Tromberg, Opt Lett 30, 1354 (Jun 1, 2005).
41. S. Ahn, A. J. Chaudhari, F. Darvas, C. A. Bouman, R. M. Leahy, Phys Med Biol 53, 3921 (Jul 21, 2008).
42. R. Sodian et al., Ann Thorac Surg 85, 2105 (Jun, 2008).
43. L. Heller, L. S. Levin, B. Klitzman, Plastic & Reconstructive Surgery 107, 1739 (2001).
44. W. L. Hickerson, S. L. Colgin, K. G. Proctor, Plastic & Reconstructive Surgery 86, 319 (1990).
45.T. P. Sullivan, W. H. Eaglstein, S. C. Davis, P. Mertz, Wound Repair and Regeneration 9, 66 (2001).
46. G. M. Morris, J. W. Hopewell, Cell Tissue Kinetics 23, 271 82 (1990).
47. W. Meyer, R. Scharz, K. Neurand, Curr Probl Dermatol 7, 39 52 (1978).
48. N. J. Vardaxis, T. A. Brans, M. E. Boon, R. W. Kreis, L. M. Marres, J Anat 190, 601 11 (1997).
49.W. Heinrich, P. M. Lange, T. Stirz, C. lancu, E. Heidermann, FEBS Letters 16, 637(1971).
50. H. Q. Marcarian, C. M.L., Am J Vet Res 27, 765 72 (1966).
51. S. Roy et al., Physiol Genomics (Mar 17, 2009).
52.S.L. Jacques, M.R. Ostermeyer, L.V. Wang, and D.V. Stephens, Proc. SPIE, 2671: p. 199-210 (1996).
53.S. Kuthirummal and N. SK., ACM Trans on Graphics, (2006).

### OTHER EMBODIMENTS

It is to be understood that while the embodiments have been described in conjunction with the detailed description thereof, the foregoing description is intended to illustrate and not limit the scope of the invention. Embodiments include a wearable goggle system for quantitative wound imaging. Although the disclosed embodiments are directed to wound oxygenation imaging, the proposed goggle system represents an enabling technology for quantitative assessment of multiple parameters essential for successful wound healing. These parameters include wound margin, perfusion, infection, angiogenesis, metabolism, and the expression of various molecular biomarkers.

## Claims

1. A system for visual wound assessment on a subject tissue, comprising:
an illumination unit adapted to emit an excitation light on the subject tissue;
an eyewear unit comprising:
an imaging unit to collect light from the subject tissue; and
an image display unit for displaying a hyperspectral image; and
a control unit operably connected to the eyewear unit and the illumination unit, the control unit comprising:
a light processing module;
an imaging fiber guide to transport emitted light received by the imaging unit to the light processing module; and
a computer programmed to:
synchronize activities of the illumination unit, the imaging unit, the light processing module, and the image display unit; and
generate the hyperspectral image in about real time.

2. The system of claim 1, further comprising:
a backpack containing at least a portion of the control unit.

3. The system of claim 1, wherein:
the illumination unit comprises in alignment:
a spectrally tunable light source; and
a programmable mirror galvanometer adapted to receive and
direct excitation light in a point-to-point scanning pattern.

4. The system of claim 1, wherein:
the illumination unit comprises in alignment:
a spectrally tunable light source;
a polarizer;
an optical chopper; and
a shutter.

5. The system of claim 4, wherein:
the illumination unit further comprises in alignment:
a programmable mirror galvanometer adapted to receive and
direct excitation light in a point-to-point scanning pattern.

6. The system of claim 1, wherein:
the illumination unit comprises in alignment:
a tunable light source; and
a lens assembly positioned to expand the light for full-field illumination of the subject tissue.

7. The system of claim 3, further comprising:
a cylindrical mirror positioned between the eyewear unit and the subject tissue.

8. The system of claim 3, wherein:
the illumination unit is positioned to provide the excitation light at an oblique angle relative to the subject tissue.

9. The system of claim 1, wherein:
the illumination unit comprises in alignment:
a tunable light source; and
a programmable mirror galvanometer adapted to receive and direct
excitation light in a point-to-point scanning pattern,
the imaging unit comprises an automated zoom lens; and
the light processing module comprises a CCD camera equipped with a liquid crystal tunable filter and an optical adaptor.

10. The system of claim 9, wherein:
the light processing module further comprises a polarizer.

11. The system of claim 9,
wherein the eyewear unit further incorporates the illumination unit.

12. The system of claim 11, further comprising:
a cylindrical mirror positioned between the eyewear unit and the subject tissue.

13. The system of claim 11, wherein:
the illumination unit is positioned to provide the excitation light at an oblique angle relative to the subject tissue.

14. The system of claim 1 wherein the hyperspectral image comprises reflectance spectra data for oxy and deoxy hemoglobin.

15. A method for visual wound assessment on a subject tissue, comprising the steps of:
providing an eyewear unit, an illumination unit, and a control unit;
illuminating a subject tissue with excitation light;
receiving emitted light from the subject tissue;
processing the emitted light to generate a processed hyperspectral image; and
displaying the processed hyperspectral image in near real time onto a head mounted image display unit.

16. The method of claim 15, wherein,
the illuminating step occurs via a point-to-point scanning pattern.
